Europäisches Patentamt

European Patent Office

Office européen des brevets

(19)

(11) Veröffentlichungsnummer: **0 000 185**
**B1**

(12) **EUROPÄISCHE PATENTSCHRIFT**

(21) Anmeldenummer: 78100223.3

(22) Anmeldetag: 23.06.78

(51) Int. Cl.³: **C 07 D 251/28**

(54) **Verfahren zur Herstellung fluorierter s-Triazine**

(30) Priorität: 01.07.77 DE 2729762

(43) Veröffentlichungstag der Anmeldung:
10.01.79 Patentblatt 79/01

(45) Bekanntmachung des Hinweises auf die Patenterteilung:
20.08.80 Patentblatt 80/17

(84) Benannte Vertragsstaaten:
BE CH DE FR GB NL

(56) Entgegenhaltungen:
DE - B - 1 044 091

(73) Patentinhaber: Bayer AG/Zentralbereich Patente, Marken und Lizenzen
D - 5090 Leverkusen 1, Bayerwerk (DE)

(72) Erfinder: Kysela, Ernst, Dr.
Auf dem Kamm 17
D - 5060 Bergisch-Gladbach 3 (DE)
Klauke, Erich, Dr.
Eichendorffweg 8
D - 5068 Odenthal (DE)
Schwarz, Herbert Dr.
Florastrasse 12
D - 5090 Leverkusen 3 (DE)
Dorlars, Alfons, Dr.
Mozartstrasse 32
D - 5090 Leverkusen 1 (DE)

## Verfahren zur Herstellung fluorierter Triazine

Gegenstand der Erfindung ist ein Verfahren zur Herstellung fluorierter s-Triazine durch Fluorierung chlorierter s-Triazine mit wasserfreiem Fluorwasserstoff, das dadurch gekennzeichnet ist, daß man flüssiges s-Trichlortriazin oder ein flüssiges, gemischt chloriert-fluoriertes s-Triazin insbesondere ein durch teilweise Fluorierung von s-Trichlortriazin erhaltenes Fluorierungsgemisch, bei Temperaturen von 50—160°C und Drucken von 2—30 bar, insbesondere 2—10 bar und ganz besonders 4—6 bar, umsetzt.

Die Reaktion kann diskontinuierlich oder kontinuierlich durchgeführt werden. Man erhält in Abhängigkeit von der verwendeten Menge HF gemischt-chloriert-fluorierte s-Triazine oder s-Trifluor-triazin. Im allgemeinen setzt man 0,5-10 Äquivalente HF pro Äquivalent auszutauschendes Chlor ein.

Bei Verwendung von 0,5-1 Äquivalent HF erhält man vorwiegend gemischt-chloriert-fluorierte s-Triazine, bei Verwendung von 1,3-3 Äquivalenten HF überwiegend s-Trifluortriazin.

Die Reaktion wird im Einzelnen folgendermaßen durchgeführt:

In einer Druckapparatur wird bei einer Innentemperatur von ca. 150°C und 3—5 bar Stickstoff geschmolzenes s-Trichlortriazin vorgelegt und im Verlauf von etwa 3 Stunden pro Äquivalent Chlor 0,5-10 Äquivalente HF zugepumpt.

Der während der Reaktion entstehende Chlorwasserstoff wird durch einen Rückflußkühler über ein Regelventil bei Drucken von 2—30, insbesondere von 2—10 bar, ganz besonders von 4—6 bar kontinuierlich entspannt. In einer bevorzugten Ausführungsweise läßt man mit dem Beginn der HF-Zuleitung bzw. nach Austausch von etwa 5—20 Äquivalentprozent Chlor die Innentemperatur auf 50 bis 130°C, insbesondere 90—110°C, sinken. Man behält diese Reaktionstemperatur durch Zuheizen bis Reaktionsende nach etwa drei Stunden bei, kühlt dann den Inhalt des Reaktors ab und rektifiziert das Reaktionsgemisch über eine Kolonne mit Rückflußkühler. Wasserfreie Flußsäure und alle übrigen möglichen Reaktionsprodukte lassen sich problemlos destillativ voneinander trennen.

Teilfluorierte s-Trihalogentriazine lassen sich ebenso in die Fluorierung einsetzen wie s-Trichlortriazin, sogar mit dem Vorzug der günstigen Dosiermöglichkeit, da alle teilfluorierten s-Trihalogentriazine bei Raumtemperatur flüssig sind; darüber hinaus kann man von Starttemperaturen ausgehen, die unter 150°C liegen.

Im Falle der kontinuierlichen Verfahrensweise wird zunächst wie oben geschildert verfahren. Sobald die jeweils gewünschte Reaktionstemperatur und/oder der erwünschte Umsetzungsgrad erreicht sind, wird zusätlich neben wasserfreier Flußsäure geschmolzenes s-Trichlortriazin in die Reaktionsmischung gepumpt. Dabei werden die beiden Reaktionspartner Flußsäure und s-Trichlortriazin im gewünschten Verhältnis eingesetzt. Die ebenfalls kontinuierlich abgezogene Reaktionsmischung wird in einer Kontirektifikation getrennt.

Gemischt chlorierte-fluorierte s-Triazine und s-Trifluortriazin sind bekannte Verbindungen, die sich insbesondere für die Herstellung von Reaktivfarbstoffen und von herbiziden Wirkstoffen eignen.

Aus der Deutschen Auslegeschrift 1 044 091 war es bereits bekannt, Cyanurchlorid, Cyanurbromid und Cyanurjodid zu ganz oder partiell fluorierten s-Triazinen umzusetzen. Dabei wird der Fluorwasserstoff jedoch auf festes Cyanurchlorid (Schmelzpunkt 146°C) aufgedrückt, ein Verfahren, das aus Gründen der Rührbarkeit und der Wärmeabfuhr (die Reaktion ist zu Beginn exotherm) technisch nicht realisierbar ist. Das erfindungsgemäße Verfahren weist diese Nachteile nicht auf und läßt sich leicht in technischem Maßstab durchführen.

### Beispiel 1

Ein unter 5 bar Stickstoffdruck stehendes Reaktionsgefäß mit Rückflußkühler und Regelventil wird mit 553 g geschmolzenem s-Trichlortriazin beschikt. Bei einer Innentemperatur von 150°C beginnt man 320 g wasserfreie Flußsäure über eine VA-Kapillare in die Schmelze mit einer Geschwindigkeit von ca. 130 g/h einzudosieren. Gleichzeitig stellt man die Kesselbeheizung ein und läßt die Innentemperatur von anfänglich 150°C auf 100°C fallen und hält dann bis Reaktionsende auf diesem Wert. Der entstehende HCl wird bei einem Entspannungsdruck von 6 bar kontinuierlich entspannt. Bei der angegebenen Dosiergeschwindigkeit ist nach etwa 2,5 Stunden die gesamte Flußsäure zugepumpt. Man rührt 0,5 Stunden nach, kühlt dann auf 20°C Innentemperatur ab und entspennt den Reaktor. Die Reaktionsmischung wird über eine VA-Füllkörperkolonne ($\phi$ 3,8 cm, Höhe 0,5 m, VA Netzwendeln) mit Rückflußteiler rektifiziert. Man erhält zunächst ca. 140 ml überschüssige Flußsäure zurück, anschießend 372 g s-Trifluortriazin vom Kp 72—74°C. Der Rückstand, bestehend aus s-Difluorchlortriazin kann im Kessel verbleiben und erneut in die Reaktion eingesetzt werden.

### Beispiel 2

Wei Beispiel 1, jedoch mit einem Entspannungsdruck von 15 bar. Nach dem Abdestillieren überschüssiger HF enthält das Reaktionsgemisch laut GC

| s-TFT | s-DFT | s-MFT | s-TCT |
|-------|-------|-------|-------|
| 51% | 40,4% | 7,9% | 0,6% |

(s-TFT: s-Trifluortriazin; s-DFT: s-Difluorchlortriazin; s-MFT: s-Monofluordichlortriazin; s-TCT: s-Trichlortriazin).

Die Komponenten werden durch fraktionierte Destillation isoliert

s-TFT: Kp 72—74°C; $n_D^{20}$ 1.3842
s-DFT: Kp 113—114°C; $n_D^{20}$ 1.4493
s-MFT: Kp 154°C; $n_D^{20}$ 1.5060.

### Beispiel 3

Wie Beispiel 1, jedoch werden anstelle von 320 g wasserfreier Flußsäure lediglich 160 g zudosiert.

Nach GC enthält das Reaktionsgemisch

| s-TFT | s-DFT | s-MFT | s-TCT |
|-------|-------|-------|-------|
| 4% | 52,4% | 39,2% | 4,4% |

### Beispiel 4

Wie Beispiel 1, jedoch mit einem Entspannungsdruck von 15 bar und einer Reaktionstemperatur von durchgehend 150°C. Nach GC enthält das Reaktionsgemisch

| s-TFT | s-DFT | s-MFT | s-TCT |
|-------|-------|-------|-------|
| 89,3% | 8,1% | 2,6% | ——— |

### Beispiel 5

Wie Beispiel 1, man läßt jedoch die Starttemperatur von 150°C auf 80°C Reaktionstemperatur fallen.

Nach dem Abdestillieren überschüssiger Flußsäure enthält das Reaktionsgemisch nach GC

| s-TFT | s-DFT | s-MFT | s-TCT |
|-------|-------|-------|-------|
| 92,3% | 7,7% | ——— | ——— |

## Patentansprüche

1. Verfahren zur Herstellung fluortierter s-Triazine durch Fluorierung chlorierter s-Triazine mit wasserfreiem Fluorwasserstoff, dadurch gekennzeichnet, daß man flüssiges, gemischt chloriert-fluoriertes s-Trichlortriazin bei 50—160°C und 2—30 bar umsetzt.

2. Verfahren nach Anspruch 1, dadurch gekennzeichnet, daß man bei 140—160°C fluoriert.

3. Verfahren nach Anspruch 1, dadurch gekennzeichnet, daß man in einer Schmelze von s-Trichlortriazin bei etwa 140—160°C bis zu einem Austausch von etwa 5—20 Äquivalentprozenten Chlor HF einleitet und anschließend bei Temperaturen von etwa 50—130°C, insbesondere 90—110°C, die Reaktion bis zum gewünschten Fluorierungsgrad weiterführt.

4. Verfahren nach Ansprüchen 1—3, dadurch gekennzeichnet, daß die Reaktion kontinuierlich durchgeführt wird.

## Revendications

1. Procédé de préparation de s-triazines fluorées par fluoration de s-triazines chlorées avec de l'acide fluorhydrique anhydre, caractérisé en ce qu'on fait réagir de la s-trichlorotriazine chlorée/fluorée mixte liquide à une température de 50—160°C et sous une pression de 2—30 bars.

2. Procédé suivant la revendication 1, caractérisé en ce qu'on effectue la fluoration à une tempèrature de 140—160°C.

3. Procédé suivant la revendication 1, caractérisé en ce qu'on introduit du HF dans une masse fondue de s-trichlorotriazine à une température d'environ 140—160°C jusqu'à un échange d'environ 5—20%-équivalents de chlore, puis on poursuit la réaction à des températures d'environ 50—130°C, en particulier, de 90—110°C jusqu'à ce qu'on atteigne le degré de fluoration désiré.

4. Procédé suivant l'une quelconque des revendications 1 à 3, caractérisé en ce qu'on effectue la réaction en continu.

## Claims

1. Process for the preparation of fluorinated s-triazines by the fluorination of chlorinated s-triazines with anhydrous hydrogen fluoride, characterised in that liquid mixed chlorofluoro-s-trichlorotriazine is reacted at 50—160°C and 2—30 bars.

2. Process according to Claim 1, characterised in that the fluorination is carried out at 140—160°C.

3. Process according to Claim 1, characterised in that HF is passed into a melt of s-trichlorotriazine at about 140—160°C until about 5—20 equivalent per cent of chlorine have been replaced and the reaction is then continued at temperatures of about 50—130°C, especially 90—110°C, until the desired degree of fluorination is reached.

4. Process according to Claims 1—3, characterised in that the reaction is carried out continuously.